# EUROPEAN PATENT APPLICATION

(11) **EP 2 236 037 A1**
(43) Date of publication of application: **06.10.2010**
(21) Application number: 09156753.7
(22) Date of filing: 30.03.2009
(51) Int. Cl.: A01N 63/00, A01N 63/02, A01N 63/04

(54) **Fungicide preparation**

(71) Applicant: Technische Universität Graz, 8010 Graz (AT); Forschungsholding TU Graz GmbH, 8010 Graz (AT)
(72) Inventor: Berg, Gabriele, 8010 Graz (AT); Zachow, Christin, 8010 Graz (AT); Müller, Henry, 8010 Graz (AT)
(74) Representative: Rentsch & Partner

(57) **Abstract**

A fungicide preparation comprises a fungicidally effective amount of at least one component selected from the group consisting of isolate Pseudomonas fluorescencs AB1, isolate Serratia plymuthica AB2, isolate Trichoderma reesei AF1, isolate Trichoderma viride AF2 and any mutant derived from these isolates and their hereditary information.

## Description

The present invention relates to a fungicide preparation as claimed in claim 1, to a method of controlling the fungus Rhizoctonia solani and to a method of protecting a plant from attack by Rhizoctonia solani as claimed in claim 4 and 6, respectively, and to an isolate as claimed in claim 10 which is present in the fungicide preparation.

The cosmopolitan fungus Rhizoctonia solani KÜHN (hereinbelow also: R. solani) is responsible for severe yield losses in a large number of crop plants (Anderson 1982, Sneh et al. 1996), having continually gained importance in recent years (Grosch et al. 2005). Table 1 shows the occurrence of Rhizoctonia root and crown rot of sugar beet.

**Table 1: occurrence of Rhizoctonia solani - Rhizoctonia root and crown rot in sugar beet in Europe, the USA and Chile in 2000 and 2001 (source: Büttner et al. 2003).**

| Country | Sugar beet 2001 (1000 ha) | *Rhizoctonia* infected (%) | |
|---|---|---|---|
| | | 2000 | 2001 |
| Austria | 45 | > 1 | < 1* |
| Belgium | 96 | 1 | 1.5 |
| France | 386 | 5 | 10 |
| Germany | 449 | 1.5 | 3 |
| Greece | 42 | 2.5 | 3 |
| Hungary | 68 | - | 1.5 |
| The Netherlands | 110 | 13 | 17 |
| Spain | 111 | 8 | < 8 |
| Sweden | 55 | - | < 1 |
| Turkey | 359 | < 1 | < 1 |
| USA | 600 | 12 | 35 |
| Chile | 47 | 15 | 23 |

| | | | |
|---|---|---|---|
| *2005: 5-8% (personal communication KWS SAAT AG) | | | |

R. solani is distinguished by a specific ecological behavior: it forms sclerotia which act as perennial organs and which are capable of surviving in the soil for a long time. Furthermore, it may live both as a saprophyte and as a pathogen. R. solani isolates can be divided into anastomosis groups (AGs) owing to their hyphal reaction, and these groups have a certain degree of host specificity (Ogoshi 1987). Rhizoctonia root and crown rot is caused by isolates of AG2-2IIIB. This group is distinguished by a very high virulence, temperature tolerance and a low degree of sensitivity to fungicides. Other host plants of AG2-2IIIB are maize and horseradish, in particular in Austria. The host spectrum of the pathogen comprises approximately 500 different plant species in total.

Due to the pathogen's physiology and ecology, its control is very difficult. While natural resistances in host plants are known and are the subject of breeding efforts, for example in sugar beet, they are not fully effective and, in the case of early attack, not sufficient in practice. Plants frequently use an accumulation of antifungal microorganisms in the rhizosphere by way of defense strategy against soil-borne fungi (Cook et al. 1995, Smith et al. 1999); antifungal microorganisms are also accumulated in the interior of the root, the endorhiza (Berg et al. 2005, Zachow et al. 2008).

Some studies into the use of antifungical bacteria as biological control agents (BCAs) exist already. Fungi of the genus Trichoderma have frequently been employed against R. solani (Elad et al. 1980, Harman and Björkman 1998, Lewis et al. 1998, Ahmed et al. 2003). Likewise, some rhizobacteria were utilized against Rhizoctonia: Bacillus licheniformis (Ahmed et al. 2003), Burkholderia cepacia (Cartwright and Benson 1994), Pseudomonas fluorescens (Howell and Stipanovic 1979), Chryseobacterium gleum (Krause et al. 2001), Lysobacter enzymogenes (Kilic-Ekici and Yuen 2003) and Pseudomonas and Serratia spp. div. (Faltin et al. 2004, Grosch et al. 2005). However, no study exists that is relevant for practical conditions and that identifies an effective control of Rhizoctonia solani in sugar beet.

The aim of the invention was thus to provide an alternative and effective preparation and method of controlling Rhizoctonia solani which can be employed in particular also in sugar beet plants.

The object is achieved by the fungicide preparation as claimed in claim 1 and by the method as claimed in claims 4 and 6, respectively.

The invention is based on the surprising finding that three specific bacterial isolates and two specific fungal isolates can be employed effectively against the phytopathogenic harmful organism Rhizoctonia solani Kühn (teleomorph: Thanatephorus cucumeris [A.B. FRANK] DONK). In principle, the abovementioned microorganism isolates can be employed for the protection of any host plant.

Specifically, the isolates are AB1, AB2, AF1 and AF2, which have been isolated from the cosmopolitan bacterial genera Pseudomonas and Serratia and from the fungal genus Trichoderma. These genera comprise naturally occurring plant microorganisms which interact with the plant predominantly in a positive way.

It has emerged that these isolates, and the mutants derived from the isolates and their hereditary information, are suitable for controlling R. solani, in particular in sugar beet plants. Hereditary information is understood hereinbelow as meaning in particular genes, gene sequences or the genome of the isolate or of a mutant.

The isolates have been deposited at the Deutsche Sammlung von Mikroorganismen und Zellkulturen (DSMZ) and at the TU Graz [Graz University of Technology] Strain Collection for Antagonistic Microorganisms (SCAM) with the accession data provided hereinbelow and are available for scientific purposes under the names mentioned hereinbelow (table 2).

**Table 2: Characterization of the isolates**

| No. | Species | DSMZ accession No. | SCAM* name | Origin |
|---|---|---|---|---|
| 1. | *Pseudomonas fluorescens* | DSM 22411 | AB1 | Potato endorhiza, Groß Lüsewitz, Germany Isolation: Jana Lottmann Proprietor: Gabriele Berg |
| 2. | *Serratia plymuthica* | DSM 22412 | AB2 | Potato endorhiza, Bonn, Germany Isolation: Gabriele Berg Proprietor: Gabriele Berg |
| 4. | *Trichoderma reesei* | DSM 22416 | AF1 | *Rhizoctonia* sclerotia, Rostock, Germany Isolation: Gabriele Berg Proprietor: Gabriele Berg |
| 5. | *Trichoderma viride* | DSM 22417 | AF2 | *Rhizoctonia* sclerotia, Rostock, Germany Isolation: Gabriele Berg Proprietor: Gabriele Berg |

| | | | | |
|---|---|---|---|---|
| *Strain Collection of Antagonistic Microorganisms of the TU Graz | | | | |

The protection of the plants, or the control of the fungi, is effected in accordance with the invention using a fungicide preparation which comprises a fungicidally effective amount of at least one component selected from the group consisting of the isolates AB1, AB2, AF1 and AF2.

The fungicidally effective amount of the isolate, or of the isolates combined with one another, will, as a rule, comprise living cells, dead cells, spores, pseudomycelium, pseudomycelial fragments and/or culture supernatant. The determination of the fungicidally effective amount can be carried out readily by the skilled worker.

Specifically, Rhizoctonia solani can be controlled in accordance with the invention by applying the fungicide preparation to Rhizoctonia solani or to at least one of its organs.

As mentioned above, the method according to the invention can be used in particular for controlling R. solani on sugar beet, but it may also be used for other Rhizoctonia host plants.

Furthermore, any host plant and in particular a sugar beet plant can be protected according to the invention from attack by Rhizoctonia solani by applying the above-described fungicide preparation to the plant to be protected or to a part thereof, to the propagation material, to the seed of the plant and/or into or onto the soil into which the plant is planted.

As a rule, isolates or isolate mixtures are formulated, applied by means of seed priming, or employed as a suspension in the dipping method.

In accordance with a preferred embodiment, a fungicide preparation comprising in each case different concentrations of the isolates of AB1, AB2, AF1 and AF2 is applied.

The fungicide preparation comprising the isolate or isolate mixture can also be applied by means of a nutrient, a solid or liquid medium and via a natural or artificial matrix.

The invention will be characterized in greater detail hereinbelow with reference to examples.
- Figure 1: shows a BOX-PCR pattern of P. fluorescens AB1, S. plymuthia AB2, T. reesei AF1 and T. viride AF2 in comparison with a standard (std).
- Figure 2: shows sugar beet seedlings after 22 days growth in plant growth cabinets at a photoperiod of 16 h light and 8 h dark at 22°C by addition of A) 0.85% sterile sodium chloride solution as the control, B) Trichoderma reesei AF1 and C) Pseu- domonas fluorescens AB1.

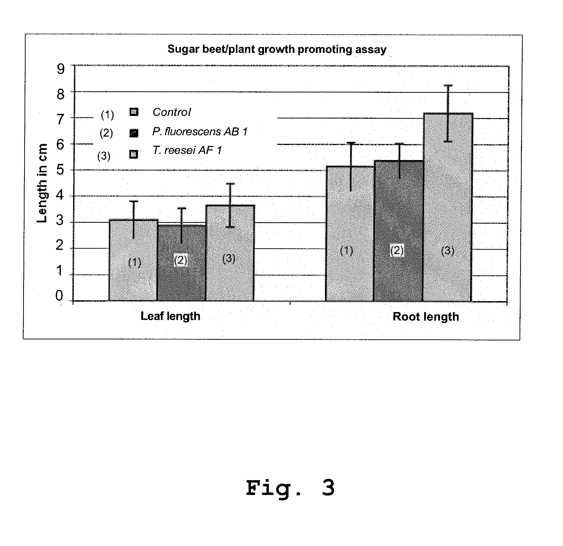

- Figure 3: shows a diagram in which the leaf lengths and the root lengths of the samples A) to C) shown in Figure 2 are plotted.
- Figure 4: shows a confocal laser scanning micrograph where A) shows the population of the rhizosphere of sugar beet seedlings with DsRed2-labeled Pseudomonas fluo- rescens AB1 cells and B) shows an enlarged section which shows the formation of aggregates (arrows) which consist of several cells.

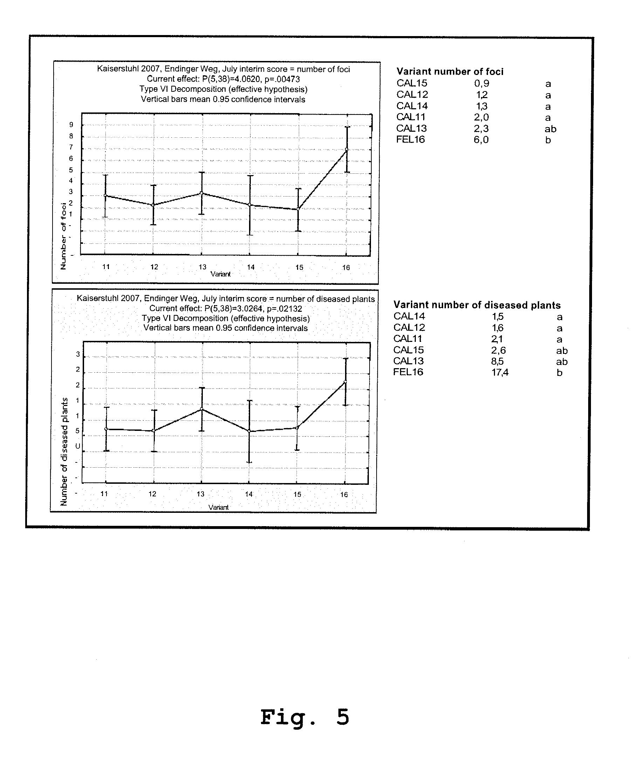

- Figure 5: is a graphic representation of the number of foci caused by R. solani in sugar beet fields (top) and the number of infected sugar beet plants specifically, where CAL = sugar beet variety CALIDA, FEL = sugar beet variety FELICITA (un- treated); 11 = control CALIDA (untreated), 12 = bacteria (for example AB1, AB2) + Trichoderma sp. (for example T. reesei AF1, T. viride AF2), 13 = bacte- rium + Trichoderma sp. (for example T. reesei AF1, T. viride AF2), 14 = bacteria (for example AB1, AB2), 15 = bacteria (for example AB1, AB2) + fat and 16 = control FELICITA.

### Example 1: Molecular fingerprint of the effective isolates

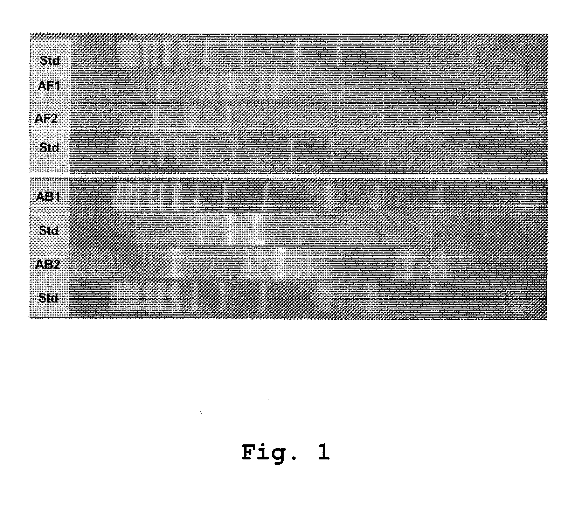

The quality, and thus the purity, of the isolates can be verified with the aid of what are known as genetic fingerprints, which can be prepared reproducibly by means of BOX-PCR. The corresponding BOX-PCR pattern is shown in Figure 1.

### Example 2: Mechanisms of action and in-vitro antifungal activity of the effective isolates

Antifungal properties are expressed by various mechanisms, for example by the production of cell wall-degrading enzymes, or the recruitment of iron by the formation of siderophores. As can be seen from tables 3 and 4, all of the isolates studied show an antifungal activity.

**Table 3: Enzymatic and plant-growth-promoting activity of the strains. The radius of the substrate degradation in a plate test, which is known to the skilled worker, is described as follows: + = 0 to 5 mm, ++ = 5 to 10 mm, +++ = > 10 mm and - = no activity (n.d. = not defined). * 1-aminocyclopropane-1-carboxylate**

| Activity | *P. fluorescens* | *S. plymuthica* | *T. reesei* AF1 | *T. viride* |
|---|---|---|---|---|
| Growth at 4°C | + | + | + | + |
| Growth at 37°C | + | + | - | - |
| Chitinases | - | + | + | - |
| Glucanases | - | - | + | + |
| Proteases | + | + | +++ | +++ |
| Indole-3-acetic acid | + | + | n.d. | n.d. |
| Siderophores | + | + | + | + |
| ACC* deaminase | + | - | n.d. | n.d. |

**Table 4: Antagonistic activity of the strains. The radius of the inhibitory zone in a dual-culture test is described as follows: + = 0 to 5 mm, ++ = 5 to 10 mm, +++ = > 10 mm and - = no inhibitory effect (n.d. = not defined). MSH = Inhibition of the microsclerotia.**

| Activity | *P. Fluorescens* AB1 | *S. Plymuthica* AB2 | *T. reesei* AF1 | *T. viride* AF2 |
|---|---|---|---|---|
| *Botrytis cinerea* | + | ++ | +++ | +++ |
| *Guignardia bidwellii* | ++ | +++ | +++ | +++ |
| *Phoma betae* | + | +++ | +++ | *n.d.* |
| *Pythium ultimum* | *n.d.* | *n.d.* +++ | | *n.d.* |
| *Rhizoctonia solani AG2-2IIIB* | +++ | +++ | +++ | +++ |
| Volatile organic compounds against *Rhizoctonia solani* | +++ | ++ | - | *n.d.* |
| *Verticilium dahliae* | +++ | +++ | +++ *MSH* | +++ *MSH* |
| *Sclerotium rolfsii* | + | +++ | + | + |

### Example 3: In-vitro plant growth promotion capacities of the effective isolates on sugar beet seedlings exemplified for P. fluorescens AB1 and T. reesei AF1

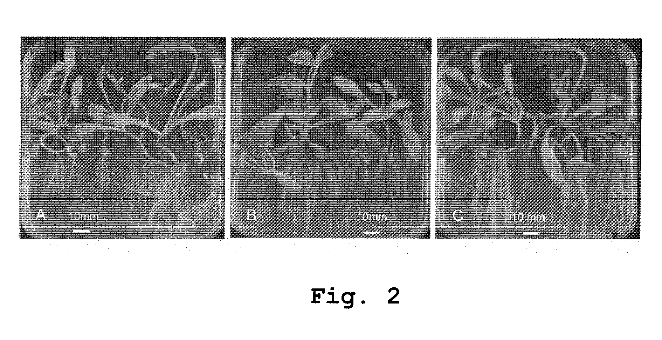

Plant growth can be promoted firstly by the suppression of pathogens, but also by the induction of resistance in the plant. However, the main criterion is that the biomass is increased. As can be seen from Figure 2, a plant-growth promoting effect has been found for both isolates studied.

### Example 4: Rhizosphere competence of the effective isolates

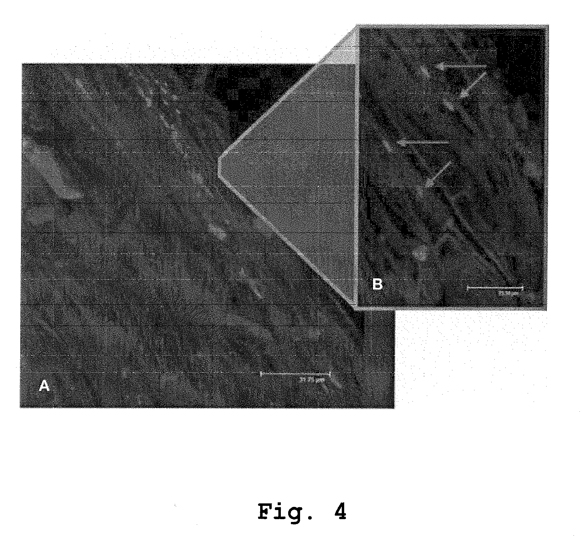

The first decisive factor in initiating the biological control activity is the adhesion of the BCAs to the root. As shown in Figure 4, the isolate according to the invention from Pseudomonas fluorescens AB1 is rhizosphere-competent.

### Example 5: Activity of the isolates under field conditions

The attack by Rhizoctonia solani was scored in July 2007 at Endingen in the Kaiserstuhl hills. As can be seen from Figure 4, markedly fewer foci, or fewer diseased plants, in comparison with the untreated control were found in the fields or plants treated with the isolates according to the invention.

### References

Ahmed AS, Ezziyyani M, Pérez Sánchez CP & Candela ME (2003): Effect of chitin on biological control activity of Bacillus spp. and Trichoderma harzianum against root rot disease in pepper (Capsicum annum) plants. Euro J Plant Pathol 109: 633-637
Anderson NA (1982): The genetics and pathology of Rhizoctonia solani. Annu Rev Phytopathol 20: 329 347
Berg G, Krechel A, Ditz M, Sikora RA, Ulrich A, Hallmann J (2005): Endophytic and ectophytic potato-associated bacterial communities differ in structure and antagonistic function against plant pathogenic fungi. FEMS Microbiol Ecol 51: 215-229
Büttner G, Pfähler B, Petersen J (2003): Rhizoctonia root rot in Europe - Indience, economic importance and concept for integrated control. Proceedings of the 1st Joint IIRB - ASSBT Congress 897-90 1
Cartwright DK & Benson DM (1994): Effect of population dynamics of Pseudomonas cepacia and Paecilomyces lilacinus on colonization of polyfoam rooting cubes by Rhizoctonia solani. Appl Environ Microb 60(8): 2852 2857
Cook RJ, Thomashow LS, Weller DM, Fujimoto D, Mazzola M, Bangera G & Kim DS (1995): Molecular mechanisms of defense by rhizobacteria against root disease. Proc Natl Acad Sci USA 92(10): 4197 4201
Elad Y, Chet I and Katan J (1980): Trichoderma harzianum: A biocontrol agent effective against Sclerotium rolfsil and Rhizoctonia solani. Phytopathology 70: 119-121
Faltin F, Lottmann J, Grosch R & Berg G (2004): Strategy to select and assess antagonistic bacteria for biological control of Rhizoctonia solani Kühn. Can J Microbiol 50: 811-820
Grosch R, Faltin F, Lottmann J, Kofoet A & Berg G (2005): Effectiveness of 3 antagonistic bacterial isolates to control Rhizoctonia solani Kühn on lettuce and potato. Canadian Journal of Microbiology 51: 345-353
Harman GE & Björkman T (1998): Potential and existing uses of Trichoderma and Gliocladium for plant disease control and plant growth enhancement. In: G.E. Harman and C.P. Kubicek (eds.), Trichoderma and Gliocladium. Vol. 2, Enzymes, biological control and commercial applications. Taylor and Francis: London, pp. 229 266
Howell CR & Stipanovic RD (1979): Control of Rhizoctonia solani in cotton seedlings with Pseudomonas fluorescens and with an antibiotic produced by the bacterium. Phytopathology 69: 480-482
Kilic-Ekici O & Yuen GY (2003): Induced resistance as a mechanism of biological control by Lysobacter enzymogenes strain C3. Phytopathology 93: 1103 1110
Krause MS, Madden LV & Hoitink HAJ (2001): Effect of potting mix microbial carrying capacity on biological control of Rhizoctonia damping-off of radish and Rhizoctonia crown and root rot of poinsettia. Biol Control 91(11): 1116 1123
Lewis JA, Larkin RP & Rogers DL (1998): A formulation of Trichoderma and Gliocladium to reduce damping-off caused by Rhizoctonia solani and saprophytic growth of the pathogen in soilless mix. Plant Disease 82: 5501-506
Ogoshi A (1987): Ecology and pathogenicity of anastomosis and intraspecific groups of Rhizoctonia solani Kühn. Annu Rev Phytopathol 25: 125 143
Smith KP, Handelsman J and Goodman RM (1999): Genetic basis in plants for interactions with disease suppressive bacteria. Proc Natl Acad Sci USA 96: 4786 4790.
Sneh B, Jabaji-Hare S, Neate S and Dijst G (1996): Non pathogenic isolates on Rhizoctonia (np-R) spp. and their role in biological control. In: Sneh B., Jabaji-Hare, S, Neate S and Dijst G., Editors, 1996. Rhizoctonia species: Taxonomy, Molecular Biology, Ecology, Pathology and Disease Control, Kluwer Academic Publishers, Dordrecht, pp. 473 484
Zachow C, Tilcher R & Berg G (2008): Sugar beet-associated bacterial and fungal communities show a high indigenous antagonistic potential against plant pathogens. Microbial Ecology 55: 119 129.

## Claims

1. A fungicide preparation comprising a fungicidally effective amount of at least one component selected from the group consisting of isolate Pseudomonas fluorescencs AB1 (DSM 22411), isolate Serratia plymuthica AB2 (DSM 22412), isolate Trichoderma reesei AF1 (DSM 22416), isolate Trichoderma viride AF2 (DSM 22417) and any mutant derived from these isolates and their hereditary information.

2. The fungicide preparation as claimed in claim 1, comprising a fungicidally effective amount of a combination comprising the isolates Pseudomonas fluorescencs AB1 (DSM 22411), Serratia plymuthica AB2 (DSM 22412), Trichoderma reesei AF1 (DSM 22416) and Trichoderma viride AF2 (DSM 22417)

3. The fungicide preparation as claimed in claim 1, comprising a fungicidally effective amount of an individual isolate selected from the group consisting of the isolates Pseudomonas fluorescencs AB1 (DSM 22411), Serratia plymuthica AB2 (DSM 22412), Trichoderma reesei AF1 (DSM 22416) and Trichoderma viride AF2 (DSM 22417).

4. A method of controlling the fungus Rhizoctonia solani, which comprises the use of the fungicide preparation as claimed in any of claims 1 to 3.

5. The use as claimed in claim 4, wherein the fungicide preparation is applied to Rhizoctonia solani or to at least one of its organs.

6. A method of protecting a plant from attack by Rhizoctonia solani, which comprises applying the fungicide preparation as claimed in any of claims 1 to 3 to the plant to be protected or a part thereof, to the propagation material, to the seed of the plant and/or into or onto the soil into which the plant is planted.

7. The method as claimed in claim 6, wherein the plant is a sugar beet plant.

8. The method as claimed in any of claims 4 to 7, wherein the fungicide preparation is applied by means of a nutrient, of a nutrient medium and/or nutrient liquid.

9. The method as claimed in any of claims 4 to 8, wherein the fungicide preparation is applied via a natural and/or artificial matrix.

10. An isolate selected from the group consisting of Pseudomonas fluorescencs AB1 (DSM 22411), Serratia plymuthica AB2 (DSM 22412), Trichoderma reesei AF1 (DSM 22416) and Trichoderma viride AF2 (DSM 22417) and any mutant derived therefrom and their hereditary information.
